# EUROPEAN PATENT SPECIFICATION

(11) **EP 2 412 321 B1**
(45) Date of publication and mention of the grant of the patent: **03.04.2013**
(21) Application number: 11175219.2
(22) Date of filing: 25.07.2011
(51) Int. Cl.: A61B 17/16

(54) **Manipulator device for a milling tool for prosthetic surgery operations**
Manipulatorvorrichtung für ein Fräswerkzeug für chirurgische Protheseoperationen
Dispositif manipulateur pour outil de fraisage pour opérations chirurgicales prothétiques

(30) Priority: 26.07.2010 IT UD20100154
(43) Date of publication of application: 01.02.2012
(73) Proprietor: HPF S.P.A., 33030 FORGARIA NEL FRIULI (UD) (IT)
(72) Inventor: Lualdi, Tommaso, 33034 Fagagna (UD) (IT)
(74) Representative: Petraz, Gilberto Luigi

(56) References cited:
- DE-A1- 2 457 713
- DE-A1-102007 019 848
- US-A1- 2003 216 716

## Description

### FIELD OF THE INVENTION

The present invention concerns a manipulator device, manual or automatic, for a milling tool for prosthetic surgery operations, used to make a bone seating, for example to install an acetabular prosthesis of the hip. In particular, the present invention concerns attachment means of the manipulator device which allow it to be selectively attached to the milling tool.

### BACKGROUND OF THE INVENTION

Milling tools in general are known, used during prosthetic surgery operations and conformed to achieve coordinated bone seatings suitable to dispose and implant relative components of surgical prostheses.

It is also known that for the correct and effective manipulation of the milling tools relative manipulator devices are provided, which can be either manual or automatic.

In any case, manipulator devices provide a central body and an attachment unit, normally made at a front end of the central body, by means of which the milling tool is operatively associated with the manipulator device.

It is known to provide attachment units that achieve a mechanical attachment of the parts, in which a male element, normally provided on the tool, cooperates due to shaped coupling with a female element, normally made on the manipulator device.

In general, known attachment units, providing an attachment/detachment only of the mechanical type, normally have disadvantages connected to the complex production and limited practicality in use, for example in snap-in systems and/or the limited precision of interconnection between the parts, for example in straight coupling systems or bayonet systems, with a consequent imprecise transmission of the operating rotation.

Therefore, current solutions of manipulator devices either have very high costs or can determine a poor quality of the action of the tool.

Moreover, although it guarantees a better transmission of the operating rotation compared with the straight coupling solution, the solution with the bayonet coupling has the disadvantage that, at the end of rotation, or in the event of a sudden drop in the rotation speed, the inertia in play can cause a counter-rotation of the tool with respect to the manipulator device and therefore, due to the very conformation of the bayonet coupling, can cause an accidental detachment of the tool from the manipulator device.

Document US-A-2003/216716 discloses a surgical tool holder for a milling tool, having a shank with a coupling end provided with a central recess and bayonet grooves for the attachment of the shank to the milling tool.

One purpose of the present invention is to achieve a manipulator device for a milling tool for prosthetic surgery operations which is simple and economic to produce and which is very precise in operations.

Another purpose of the present invention is to achieve a manipulator device which limits to a minimum the possibility of an accidental detachment of the parts.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purposes, a manipulator device for a milling tool for prosthetic surgery operations according to the present invention comprises a central body and at least attachment means angularly constrained to the central body and able to achieve the selective attachment/detachment of the central body to/from the milling tool.

According to the present invention, the attachment means are provided at one end of the central body and comprise a blind insertion seating open toward an insertion end, in an axial direction to a median axis of rotation of the central body.

The attachment means also comprise at least a semi-circular groove made radially through to the insertion seating and open toward the insertion end.

This conformation allows a mating connection element, provided on the tool, to be inserted coaxially inside the insertion seating and, once inserted, to be rotated in guided fashion for the segment defined by the semi-circular groove, substantially achieving a bayonet coupling of the parts.

According to the present invention, the attachment means also comprise a magnetic element, provided inside the insertion seating, so as to retain magnetically the connection element of the tool inside the insertion seating, contrasting the axial movement thereof at exit from the insertion seating.

In this way, and in particular the force of magnetic attraction developed by the magnetic element, retains the connection element of the tool close up to a bottom wall of the insertion seating, so as to reduce to a minimum the vibrations or reciprocal axial movements of the parts during the operating steps, and thus increase the quality of the operations made by the tool.

Moreover, in the event of a reciprocal counter-rotation of the manipulator device and the tool, for example due to the inertia, the magnetic element retains the connection element in the insertion seating.

Therefore, with the present invention, a manipulator device is obtained which has reduced production costs, is practical to use and guarantees an attachment with great stability and high quality, also preventing accidental detachments.

According to a variant, the manipulator device also comprises connection means, provided on the central body on the side opposite the attachment means, and conformed to allow the operating connection of the central body to an apparatus suitable to make the central body rotate with a determinate speed of rotation.

According to another variant, the central body comprises a handle assembled externally to the central body and rotatably unconstrained from the latter, so as to allow the manipulator device to be gripped during the rotation steps of the central body.

According to another variant, a protection element is provided, disposed inside the insertion seating to protect the magnetic element, and able to define the bottom wall of the insertion seating.

### BRIEF DESCRIPTION OF THE DRAWINGS

These and other characteristics of the present invention will become apparent from the following description of a preferential form of embodiment, given as a non-restrictive example with reference to the attached drawings wherein:

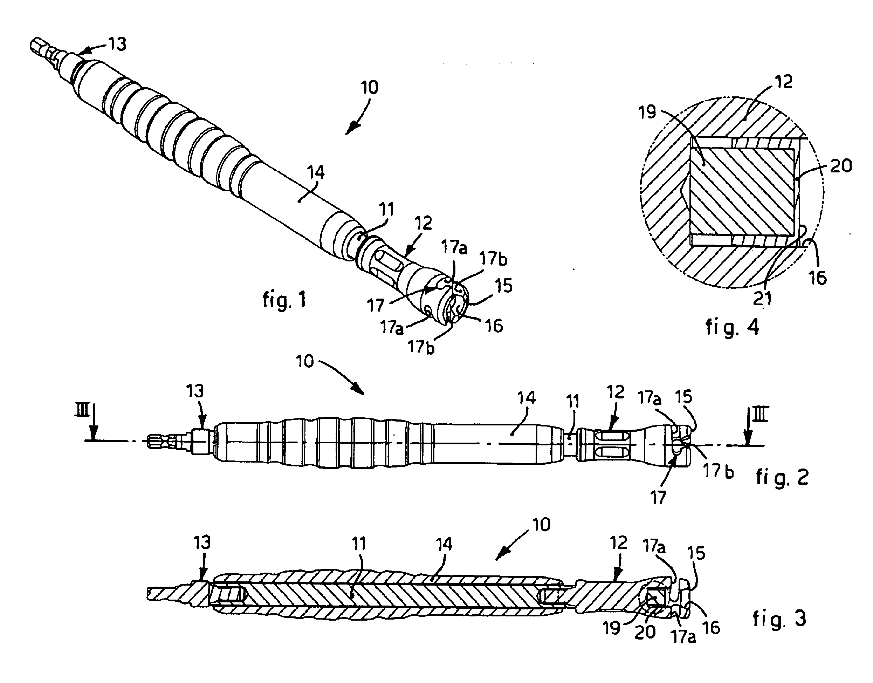

- fig. 1 is a three-dimensional view of a manipulator device for a tool according to the present invention;
- fig. 2 is a lateral view of the tool in fig. 1;
- fig. 3 is a view in section from III to III in fig. 2;
- fig. 4 is an enlarged detail of the section in fig. 3.

To facilitate comprehension, the same reference numbers have been used, where possible, to identify identical common elements in the drawings.

### DETAILED DESCRIPTION OF A PREFERENTIAL FORM OF EMBODIMENT

With reference to the attached drawings, the reference number 10 is used to denote in its entirety a manipulator device according to the present invention for a milling tool, of a substantially known type and not shown in the drawings, of the type used in prosthetic surgery operations.

In particular, the manipulator device 10 comprises a central body 11, in this case oblong and substantially cylindrical, an attachment unit 12 by means of which the manipulator device 10 can be selectively associated with a relative milling, drilling or other tool, and a connection unit 13, by means of which the manipulator 10 can be selectively connected to a relative rotation apparatus, not shown in the drawings.

The central body 11 comprises a handle 14 made of self-lubricating plastic material, such as for example Teflon®, and assembled externally to the central body 11.

In particular, the handle 14 is mounted on the central body 11 so as to be both constrained axially to a median axis of rotation, and also rotatably unconstrained with respect to the central body 11.

The handle 14 also has an anatomical surface conformation to allow a correct and efficient grip of the manipulator device 10 during the rotation steps of the central body 11.

In this case, both the attachment unit 12 and the connection unit 13 are mounted by screwing at respective opposite ends of the central body 11, in a condition angularly constrained and substantially coaxial with the median axis of rotation of the latter.

The connection unit 13 is of the rapid attachment type and, depending on the power transmission of the rotation apparatus, may be chosen from among those available on the market, of the mechanical, pneumatic, electronic or other type.

The attachment unit 12 comprises a front end 15, in which an insertion seating 16 and a guide groove 17 are made.

The insertion seating 16 is made blind in an axial direction to the median axis of rotation of the central body 11, and is open toward the front end 15.

The guide groove 17 comprises, specularly, two semi-circular segments 17a made radially through to the insertion seating 16, and two continuous linear segments 17b at one end of the relative semi-circular segment 17a, open toward the front end 15 and substantially parallel to the median axis of rotation of the central body 11.

This conformation allows to insert a mating connection element of the tool, advantageously made of ferromagnetic material, which is inserted with an axial movement into the insertion seating 16 following the linear segments 17b, and then rotated inside the insertion seating 16 itself, following the semi-circular segments 17a. Therefore, this double movement substantially achieves a bayonet coupling between the parts.

Inside the insertion seating 16 a magnet 19 is positioned, in this case a natural magnet, disposed so as to generate a magnetic field whose field lines tend to generate an axial magnetic force of traction toward the inside of the insertion seating 16.

However, it cannot be excluded that, in other solutions, the magnet 19 can be of the electronically commanded or other type, while still generating an axial magnetic force of traction toward the inside of the insertion seating 16.

A protection cap 20 is also provided inside the insertion seating 16, disposed to cover the magnet 19, to protect it from knocks.

On one side the protective cap 20 determines with its thickness a positioning compensation for the magnet 19 and on the other side defines a bottom wall 21 of the insertion seating 16.

It is clear that modifications and/or additions of parts may be made to the manipulator device 10 as described heretofore, without departing from the field and scope of the present invention.

It is also clear that, although the present invention has been described with reference to some specific examples, a person of skill in the art shall certainly be able to achieve many other equivalent forms of manipulator device for a milling tool for prosthetic surgery operations, having the characteristics as set forth in the claims and hence all coming within the field of protection defined thereby.

## Claims

1. Manipulator device for a milling tool for prosthetic surgery operations, comprising a central body (11) and at least attachment means (12) angularly constrained to said central body (11) and able to achieve selective attachment/detachment of said central body (11) to/from the milling tool, the attachment means (12) being provided at one end of said central body (11) and comprising mechanical attachment means comprising an insertion seating (16), blind and open toward an insertion end (15) in a direction axial to a median axis of rotation of said central body (11) and at least a groove (17) made radially through to the insertion seating and open toward said insertion end (15), **characterized in that** the attachment means (12) comprise magnetic attachment means comprising a magnetic element (19) provided inside said insertion seating (16), and able to magnetically hold a connection element of the milling tool inside said insertion seating (16).

2. Device as in claim 1, **characterized in that** the mechanical attachment means define a bayonet joint.

3. Device as in claim 2, **characterized in that** the guide groove (17) comprises, specularly, two semi-circular segments (17a) made radially through to the insertion seating (16), and two continuous linear segments (17b) at one end of the relative semi-circular segment (17a), open toward the insertion end (15) and substantially parallel to the median axis of rotation of the central body (11).

4. Device as in any claim hereinbefore, **characterized in that** it also comprises connection means (13) provided on the central body (11) on the opposite side to the attachment means (12), and conformed to allow the operating connection of said central body (11) to an apparatus suitable to make said central body (11) rotate.

5. Device as in any claim hereinbefore, **characterized in that** the central body (11) comprises a handle (14) assembled externally to said central body (11) and rotatably unconstrained from the latter.

6. Device as in any claim hereinbefore, **characterized in that** it comprises a protection element (20) disposed inside the insertion seating (16) to protect the magnetic element (19), and able to define a bottom wall (21) of said insertion seating.

7. Device as in any claim hereinbefore, **characterized in that** the magnetic element (19) is a natural magnet.

## Patentansprüche

1. Manipulationsvorrichtung für ein Fräswerkzeüg für chirurgische Protheseoperationen, welche einen Mittenkörper (11) und zumindest ein Befestigungselement (12), welches am Mittenkörper (11) angewinkelt gehalten ist und dazu in der Lage ist, eine selektive Befestigung bzw. Entnahme des Mittenkörpers (11) an bzw. vom Fräswerkzeug zu erzielen, enthält, wobei das Befestigungselement (12) an einem Ende des Mittenkörpers (11) bereitgestellt ist und ein mechanisches Befestigungselement enthält, welches einen Einstecksitz (16), welcher in Richtung zu einem Einsteckende (15) in einer Richtung axial zu einer mittleren Drehachse des Mittenkörpers (11) geschlossen und geöffnet ist, und zumindest eine Nut (17) enthält, welche radial durchgängig zum Einstecksitz erstellt ist und in Richtung zum Einsteckende (15) geöffnet ist, **dadurch gekennzeichnet, dass** das Befestigungselement (12) ein magnetisches Befestigungselement enthält, welches ein Magnetelement (19) enthält, welches innerhalb des Einstecksitzes (16) bereitgestellt ist und dazu in der Lage ist, ein Verbindungselement des Fräswerkzeugs innerhalb des Einstecksitzes (16) magnetisch zu halten.

2. Vorrichtung nach Anspruch 1, **dadurch gekennzeichnet, dass** das mechanische Befestigungselement eine Bajonettverbindung bestimmt,

3. Vorrichtung nach Anspruch 2, **dadurch gekennzeichnet, dass** die Führungsnut (17), zueinander spiegelförmig, zwei halbrunde Segmente (17a), welche radial durchgängig zum Einstecksitz (16) erstellt sind, und zwei durchgängig lineare Segmente (17b) an einem Ende des jeweiligen halbrunden Segments (17a) enthält, welche in Richtung zum Einsteckende (15) geöffnet sind und zur mittleren Drehachse des Mittenkörpers (11) im Wesentlichen parallel sind.

4. Vorrichtung nach einem der vorhergehenden Anspruche, **dadurch gekennzeichnet, dass** sie ebenso ein Verbindungselement (13) enthält, welches an der gegentiberliegenden Seite zum Befestigungselement (12) am Mittenkörper (11) bereitgestellt ist, und dazu ausgelegt ist, die betriebsmäßige Verbindung des Mittenkörpers (11) mit einer Einrichtung zu ermöglichen, welche zur Umdrehung des Mittenkörpers (11) geeignet ist.

5. Vorrichtung nach einem der vorhergehenden Ansprüche, **dadurch gekennzeichnet, dass** der Mittenkörper (11) ein Griffstück (14) enthält, welches extern zum Mittenkörper (11) zusammengesetzt ist und zu diesem ungehindert umdrehbar ist.

6. Vorrichtung nach einem der vorhergehenden Ansprüche, **dadurch gekennzeichnet, dass** sie ein Schutzelement (20) enthält, welches innerhalb des Einstecksitzes (16) angeordnet ist, um das magnetische Element (19) zu schützen, und dazu ausgelegt ist, eine untere Wand (21) des Einstecksitzes zu bestimmen.

7. Vorrichtung nach einem der vorhergehenden Ansprüche, **dadurch gekennzeichnet, dass** das magnetische Element (19) ein natürlicher Magnet ist.

## Revendications

1. Dispositif manipulateur pour un outil de fraisage pour opérations chirurgicales prothétiques comprenant un corps central (11) et au moins des moyens de fixation (12) angulairement fixés au dit corps central (11) et capable de permettre la fixation/le détachement sélectif dudit corps central (11) à l'outil de fraisage/de l'outil de fraisage, les moyens de fixation (12) étant fourni au niveau d'une extrémité dudit corps central (11) et comprenant des moyens de fixation mécanique comprenant un siège d'insertion (16), aveugle et ouvert en direction d'une extrémité d'insertion (15) dans une direction axiale par rapport à un axe médian de rotation dudit corps central (11) et au moins une rainure (17) formé radialement dans le siège d'insertion et ouvert en direction de ladite extrémité d'insertion (15), **caractérisé en ce que** les moyens de fixation (12) comprennent des moyens de fixation magnétique comprenant un élément magnétique (19) fourni à l'intérieur dudit siège d'insertion (16), et capable de magnétiquement maintenir un élément de raccordement de l'outil de fraisage à l'intérieur dudit siège d'insertion (16).

2. Dispositif selon la revendication 1, **caractérisé en ce que** les moyens de fixation mécanique définissent un joint à baïonnette.

3. Dispositif selon la revendication 2, **caractérisé en ce que** la rainure de guidage (17) comprend, de manière spéculaire, deux segments semi-circulaires (17a) formés radialement dans le siège d'insertion (16), et deux segments linéaires continus (17b) au niveau d'une extrémité du segment semi-circulaire relatif (17a), ouverts en direction de l'extrémité d'insertion (15) et sensiblement parallèles à l'axe médian de rotation du corps central (11).

4. Dispositif selon l'une quelconque des revendications précédentes, **caractérisé en ce qu'**il comprend également un moyen de raccordement (13) fourni sur le corps central (11) sur le côté opposé aux moyens de fixation (12), et configuré pour permettre le raccordement fonctionnel dudit corps central (11) à un appareil adapté à faire pivoter ledit corps central (11).

5. Dispositif selon l'une quelconque des revendications précédentes, **caractérisé en ce que** le corps central (11) comprend une poignée (14) assemblée par l'extérieur au dit corps central (11) et non contrainte en rotation par rapport à ce dernier.

6. Dispositif selon l'une quelconque des revendications précédentes, **caractérisé en ce qu'**il comprend un élément de protection (20) disposé à l'intérieur du siège d'insertion (16) pour protéger l'élément magnétique (19) et capable de définir une paroi inférieure (21) dudit siège d'insertion.

7. Dispositif selon l'une quelconque des revendications précédentes, **caractérisé en ce que** l'élément magnétique (19) est un aimant naturel.
